# DEMANDE DE BREVET EUROPEEN

(11) **EP 2 359 690 A1**
(43) Date de publication de la demande: **24.08.2011**
(21) Numéro de dépôt: 10305180.1
(22) Date de dépôt: 23.02.2010
(51) Int. Cl.: A01N 25/04, A01N 65/00, A61K 47/02, A61K 36/61, A61K 36/752, A61K 36/54, A61K 36/53, A01N 59/10, A01N 59/16, A01N 25/28, A01P 1/00, A61K 9/127

(54) **Multiplication de l'efficacité des agents anti-infectieux par une composition comprenant conjointement un agent dispersant et un agent activateur métallique**

(71) Demandeur: East Coast Pharmaceutical Research LLC, New York, NY 10022 (US)
(72) Inventeur: Lagny, Pierre, Celbridge, Co. Kildare (IE); Herault, Jean-Jacques, Plaistow, London E130LJ (GB)
(74) Mandataire: Lemoine, Jean-Sébastien

(57) **Abrégé**

La présente invention concerne une composition inhibitrice ou destructrice d'au moins un être vivant ou unicellulaire, qui comprend au moins un agent anti-infectieux et au moins un agent activateur comportant au moins un élément métallique. La présente invention concerne également l'utilisation d'une telle composition.

## Description

La présente invention concerne de manière générale la destruction et/ou l'inhibition des êtres vivants ou unicellulaires tels que les protozoaires, les microbes, les bactéries, les gamètes, les champignons, les levures, les parasites ou autres.

On connaît déjà de nombreuses substances inhibant ou détruisant les êtres vivants unicellulaires, et parmi elles les agents tensio-actifs tels que les sels d'ammonium quaternaire. En particulier on sait que les halogénures d'ammonium quaternaire tels que le chlorure de benzalkonium (ou chlorure d'alkyl-benzyl-diméthyl-ammonium), seuls ou en combinaison avec d'autres principes actifs, sont avantageux dans ces applications (voir par exemple les brevets anglais GB1 554 615, les brevets français FR 2431 859, FR 2 483 177, FR 2 379 508, FR 2 384 497, FR 2 457 641, FR 2 573 624, FR 2 418 221, FR 2 562 888, les brevets européens EP 0 243 713, EP 0 175 338, EP 0 132 963, EP 0 127 131, EP 0 094 562, EP 0 076 136, EP 0 068 399, EP 0 037 593, et les demandes internationales WO 84/00877 et WO 84/02649).

Par ailleurs, l'art antérieur a déjà fourni de nombreux procédés de fabrication de ces sels d'ammonium quaternaires (parmi les halogénures d'ammonium quaternaires), directement applicables aux chlorures et/ou iodures, et/ou bromures, et/ou chloro-iodites. (cf. les brevets ci-dessus et également les brevets français FR 2 472 558, FR 2 033 044 et européens EP 0 094 552 et EP 0 012 296).

On connaît également le fluorure d'ammonium et ses procédés de préparation et purification (voir par exemple les brevets français FR 2 244 713, 2 253 710 et européen EP 0 002 016), et des sels d'ammonium quaternaires perfluorés ou polyfluorés (par exemple brevets français 2 038 421, 2 051 095, 2 153 489 et européens EP 0 073 760, EP 0 100 478, EP 0 100 477, et EP 0149 172).

Le fluor ionique est par ailleurs bien connu pour ses propriétés anti-caries dans les applications dentaires (par exemple brevet américain 4 473 547), éventuellement associé à un ammonium quaternaire cationique (voir brevets français 1 486 676 et 1 297 708). Dans ces derniers documents on met en évidence l'avantage d'associer dans un même composé d'une part le fluor connu pour ses propriétés anti-caries, et d'autre part les sels d'ammonium quaternaire tensio-actifs connus pour leurs propriétés bactéricides. Cependant aucune activité véritablement synergique n'est démontrée dans ces applications pour les dentifrices, avec les fluorures d'ammonium quaternaire testés que ce soit vis-à-vis des propriétés anti-caries ou vis-à-vis des propriétés bactéricides.

Le brevet français FR 1 297 708 décrit en particulier le fluorure de lauryl-benzyl-diméthyl-ammonium, un procédé de préparation de ce fluorure et son application dans une pâte dentifrice.

Le brevet italien FR 1 153 530 décrit un procédé de préparation de carbonates d'ammonium quaternaires puis, à partir de ces carbonates, des halogénures d'ammonium quaternaires par échange d'anions avec l'acide correspondant plus fort que l'acide carbonique. Cependant, si les composés obtenus grâce à ce procédé sont aptes à être appliqués dans la désinfection de surfaces et dans l'industrie, ils ne peuvent être directement utilisés sur l'être humain ou animal, par exemple dans des compositions pharmaceutiques dans lesquelles les composés doivent avoir une grande pureté.

Dans le brevet européen EP 0308564, est posé le problème de fournir une composition totalement inhibitrice ou destructrice d'êtres vivants unicellulaires et qui soit simultanément applicable à l'être vivant, humain, animal ou végétal sans provoquer pour autant des réactions secondaires néfastes. La solution à ce problème décrite dans EP 0308564 consiste en une composition renfermant au moins un fluorure de myristalkonium, qui peut être utilisée pour l'être humain ou animal. Toutefois, cette composition présente l'inconvénient d'une irritation au point d'injection, sauf lors de l'utilisation d'une voie d'administration centrale. Cette irritation, causée par la quantité nécessaire de principe actif dans la composition, peut être améliorée par la diminution de la quantité de ce principe actif lorsqu'il est associé à un agent activateur et à un agent dispersant.

Par ailleurs, le brevet européen EP 0310476 décrit une composition pharmaceutique renfermant du fluor ionique et ionisable et du lithium ionique ou ionisable, notamment du fluorure de lithium. Toutefois, cette composition présente l'inconvénient de ne pas utiliser un dispersant et donc de rendre plus difficile la relation de la composition avec l'agent infectieux, ce qui est modifié radicalement par l'adjonction de l'agent dispersant.

Les compositions décrites dans les brevets de l'art antérieur ci-dessus mentionnés nécessitent cependant des teneurs suffisamment élevées en agents anti-infectieux pour être efficaces. Mais ces quantités élevées ont pour inconvénient majeur de provoquer des effets indésirables sur les êtres humains et animaux.

La demanderesse a découvert de manière surprenante que l'action conjointe d'un ou plusieurs agents activateurs comportant au moins un élément métallique (notamment sous forme d'ions, de sels ou de colloïdes métalliques, de type nanoparticulaire ou pas) et d'agents dispersants liposomiques, ou micellaires, ou peptidiques, ou sous forme de micro-émulsion ou de nanoémulsion, ou sous forme de microcapsule, dans une composition comportant un agent anti-infectieux, permet d'en augmenter le pouvoir inhibiteur ou destructeur d'êtres vivant ou unicellulaires.

Par contre, l'association en tant que telle d'un tel agent dispersant et d'un activateur métallique n'a pas en elle-même un pouvoir inhibiteur ou destructeur d'une infection, et agit seulement comme catalyseur des agents anti-infectieux utilisés (encore appelées principes actifs dans la présente demande). Cela a pour conséquence directe que la quantité nécessaire pour inhiber ou détruire des êtres vivants ou unicellulaires est considérablement diminuée par rapport à celle généralement nécessaire en l'absence de ces catalyseurs (agent(s) activateur(s) métallique(s) et agent dispersant utilisé conjointement).

En outre, la demanderesse a découvert que l'élément métallique de l'agent (ou des agents) activateur(s) doit être présent dans la composition à raison d'au plus 20 ppm ou mg/L de composition. En effet, des doses supérieures à 20 ppm ou mg/L conduisent de manière surprenante à un effet inverse, c'est à dire à une diminution du pouvoir inhibiteur et/ou destructeur des principes actifs.

Plus particulièrement, la présente invention a donc pour objet une composition inhibitrice et/ou destructrice d'au moins un être vivant ou unicellulaire, qui comprend :
- au moins un agent anti-infectieux, et
- au moins un agent activateur comportant au moins un élément métallique.

Selon l'invention, la composition comporte en outre conjointement au moins un agent dispersant liposomique ou micellaire, ou peptidique, ou sous forme de micro-émulsion ou de nanoémulsion, ou sous forme de microcapsule, et l'élément métallique de l'agent activateur est présent dans ladite composition à raison d'au plus 20 mg/L.

### Agent dispersant

Les agents dispersants sont connus et utilisés depuis des années mais aucun n'a été utilisé conjointement avec des ions métalliques pour augmenter l'efficacité d'un agent anti-infectieux.

L'action d'agents dispersants liposomiques sur des agents anti-infectieux est connue de l'homme de l'art, comme l'attestent les publications de Ravaoarinoro M. : 1. *"*Pharmacokinetics of cationic liposome-encapsuled doxycycline in mice challenged with genital infection by Chlamydia trachomatis" de Selliah S., Ravaoarinoro M, dans Chemotherapy, 2004 Apr, 50 (1), 17-21. PMID: 15084800 [PubMed - indexed for Medline] ; 2. « In vitro inhibition of Chlamydia trachomatis growth by liposome-encapsuled cyclines" de Sangaré L., Morisset R., Ravaoarinoro M, dans Pathol Biol (Paris), 2001 Feb, 49 (1), 53-6. French, PMID: 11265224 [PubMed - indexed for Medline] ; 3. *"*Effets of cationic liposome-encapsuled doxycycline on experimental Chlamydia trachomatis genital infection in mice." de Sangaré L., Morisset R., Gaboury L., Ravaoarinoro M., dans J Antimicrob Chemother., 2001 Mar, 47 (3), 323-31.PMID: 11222565 [PubMed - indexed for Medline] ; 4. *"*In-vitro anti-chlamydial activities of free and liposomal tetracycline and doxycycline" de Sangaré L., Morisset R., Ravaoarinoro M., dans J Med Microbiol., 1999 Jul, 48 (7), 689-93. PMID: 10403420 [PubMed - indexed for Medline] ; 5. *"*Incorporation rates, stabilities, cytotoxicities and release of liposomal tetracycline and doxycycline in human serum" de Sangaré L., Morisset R., Omri A., Ravaoarinoro M., dans J Antimicrob Chemother., 1998 Dec, 42 (6), 831-4.PMID: 10052911 [PubMed - indexed for Medline] ; 6. *"*Comparison of the bactericidal action of amilkacin, netilmicin and tobramycin in free and liposomal formulation against Pseudomonas aeruginosa.", de Omri A., Ravaoarinoro M., in Chemotherapy, 1996 May-Jun, 42 (3), 170-6. PMID: 8983883 [PubMed - indexed for Medline] ; 7. *"*Incorporation, release and in-vitro antibacterial activity of liposomal aminoglycosides against Pseudomonas aeruginosa.", de Omri A., Ravaoarinoro M., Poisson M., dans J Antimicrob Chemother., 1995 Oct, 36 (4), 631-9. PMID: 8591937 [PubMed - indexed for Medline] ; 8. "Liposomes, in the treatment of infections", de Ravaoarinoro M., Toma E, dans Ann Med Interne (Paris),1993, 144 (3), 182-7. Review. French. PMID: 8368703 [PubMed - indexed for Medline] ; 9. *"*Efficient entrapment of amikacin and teicoplamin in liposomes. ", de Ravaoarinoro M., Toma E., Agbaba O., Morisset R., dans J Drug Target., 1993, 1 (3), 191-5. PMID: 8069560 [PubMed - indexed for Medline].

Toutefois, dans ces publications, l'agent dispersant liposomique n'est pas associé à un agent activateur métallique, comme dans la composition selon l'invention et cela présente l'inconvénient de ne pas apporter l'activité surprenante de la présente invention et de ne pas comporter son caractère universel, c'est-à-dire non seulement sur l'humain mais dans l'ensemble des règnes animal et végétal.

L'agent dispersant utilisable dans la composition selon l'invention peut être liposomique, ou micellaire, ou peptidique, ou sous forme de micro-émulsion ou de nanoémulsion, ou sous forme de microcapsule.

L'agent activateur ne peut pas être présent dans la composition selon l'invention à des concentrations supérieures à 20 ppm (ou mg/L). Ce n'est que dans ces concentrations précises allant de 0 à 20 ppm que l'on observe les effets surprenants précédemment décrits et revendiqués dans la présente invention (c'est-à-dire une activation sensible du pouvoir inhibiteur du (ou des) principes actifs présents dans la composition selon l'invention

Dans le cas d'un agent dispersant liposomique, celui-ci peut être avantageusement choisi parmi les liposomes naturels ou synthétiques, de type anionique ou cationique.

Cet agent dispersant liposomique a pour première fonction de réduire la tension superficielle afin d'émulsionner les divers principes actifs (ou agents anti-infectieux), eux-mêmes associés à des agents activateurs métalliques.

Cet agent dispersant liposomique a pour deuxième fonction d'envelopper à l'intérieur de sa microsphère lipidique les principes actifs associés aux adjuvants activateurs métalliques.

Dans les d'un agent dispersant micellaire, le principe actif n'est pas dans une vésicule, mais inséré entre deux micelles.

Dans le cas d'un agent dispersant peptidique, celui-ci constitue un vecteur auquel la molécule active est liée.

Dans le cas de la microcapsule, le principe actif peut être enrobé, dans un alginate par exemple.

### Agent activateur

L'agent activateur de la composition selon l'invention comporte au moins un élément métallique, qui est unitaire ou associé, mais jamais sous forme de combinaison, c'est-à-dire ne formant pas une molécule avec le principe actif.

Ainsi, l'agent activateur peut comporter un seul élément métallique unitaire ou une association de plusieurs éléments métalliques unitaires, qui ne sont pas combinés de manière à former une nouvelle molécule, conformément à la définition ci-dessus.

L'élément métallique de l'agent activateur dans la composition selon l'invention peut être constitué de tous les métaux connus et répertoriés au tableau périodique de Mendeleïev.

On citera plus particulièrement à titre d'élément métallique pouvant avantageusement entrer dans la composition de l'élément activateur les éléments suivants : lithium, sodium, cadmium, cobalt, cuivre, zinc et étain, ou autres.

L'agent activateur de la composition selon l'invention peut se présenter sous différentes formes, par exemple sous forme d'un colloïde métallique nano-particulaire ou non, ou d'un sel métallique, ou sous forme d'un ion métallique, ou sous la forme d'un composé naturel contenant déjà un ensemble de métaux comme l'eau de mer désodée et déshydratée.

Parmi les agents activateurs utilisables dans le cadre de la présente invention, on citera plus particulièrement et de façon non limitative les fluorures et tout autre sel de lithium, de sodium, d'étain, de cadmium, de cobalt, de cuivre, de zinc, etc.

### Agent anti-infectieux

La composition selon l'invention peut s'appliquer normalement à l'ensemble des principes actifs (ou agents) anti-infectieux existants quelle que soit leur origine, naturelle ou chimique de synthèse, et en particulier aux agents anti-infectieux suivants : la totalité des bactéricides, des antibiotiques, des fongicides, des virucides, des antiparasitaires, des désinfectants de surface, des spermicides, et des phytosanitaires, qu'ils soient d'origine chimique, végétale ou naturelle.

A titre d'antibiotiques utilisables dans le cadre de la présente invention, on citera plus particulièrement les pénicillines, les céphalosporines, les cyclines, les aminosides, les macrolides, les sulfamides, les quinolones, les phénicolés, etc.

A titre d'antibiotiques utilisables dans le cadre de la présente invention, on peut encore citer les lincosamides, les synergistines, les antibiotiques glycopeptidiques, l'acide fusidique, la fosfomycine, la rifampicine, etc.

A titre de virucides utilisables dans le cadre de la présente invention, on peut notamment citer :
- les virucides chimiques, qui peuvent être choisis parmi les ammoniums quaternaires, par exemple le chlorure de benzalkonium, et le fluorure de tétradécyl-diméthyl-benzyl-ammonium (ou fluorure de myristalkonium), etc. et
- les virucides d'origine végétale, qui peuvent être choisis parmi les huiles essentielles d'Origan (*Origanum majorana*)*,* de Thym (*Thymus vulgaris*)*,* et de Sarriette (*Satureja montana*)*,* etc., ou parmi les extraits de plantes comme un extrait de pépin de pamplemousse (*Citrus paradisi*)*,* ou
- les virucides d'origine naturelle choisi parmi les produits d'origine naturelle comme la Propolis, etc.

A titre de bactéricides utilisables dans le cadre de la présente invention, on peut notamment citer les bactéricides chimiques, tels que les ammoniums quaternaires, les biguanides, les carbanilides, les composés phénoliques, les composés chlorés, et le glutaraldehyde, etc.

Selon un mode de réalisation particulièrement avantageux de la présente invention, l'agent anti-infectieux est l'ammonium quaternaire fluoré suivant : le fluorure de tétradécyl-diméthyl-benzyl-ammonium (ou FTDBA, ou fluorure de myristalkonium). Avec un tel agent anti-infectieux présent dans la composition selon l'invention, cette dernière peut être administrable par injection.

Le FTDBA appartient à la famille des ammoniums quaternaires cationiques connus pour ses propriétés antibactériennes puissantes, parmi lesquels le chlorure de benzalkonium ou chlorure d'alkyl-diméthyl-benzyl-ammonium est l'un des plus actifs. L'action du FTDBA a été décrite dans les deux publications suivantes : *"*Innocuity of tetradecyl-dimethyl-benzyl-ammonium fluoride on the DNA of human spermatozoa", de Laforest G., Sergerie M., Bleau G, dans Contraception, 2004 May; 69 (5) : 425 - 32 (PMID : 15105067) [PubMed - indexed for Medline]; et "Efficacy of a new quaternary ammonium compound against TB", de Byrne C., Healy TM, dans Ir J Med Sci, 1999 Jan - Mar; 168 (1) : 45 - 6 (PMID : 10098344) [PubMed - indexed for Medline]. [TB = Mycobacterium tuberculosis].

A titre de fongicides utilisables dans le cadre de la présente invention, on peut notamment citer :
- les fongicides chimiques, par exemple l'econazole, le ketoconazole, le ciclopirox olamine, etc., et
- les fongicides d'origine végétale, par exemple les huiles essentielles de géranium rosat (*Pelargonium asperum*)*,* de palmarosa (*Cymbopogon martinis* variété *motia*)*,* et de lavande aspic (*Lavendula latifolia*)*,* etc., ou les extraits de plantes comme un extrait de pépin de pamplemousse (*Citrus paradisi*)*,* ou
- les fongicides d'origine naturelle comme la Propolis, etc.

A titre de spermicides utilisables dans le cadre de la présente invention, on peut notamment citer les ammoniums quaternaires (en particulier le chlorure de benzalkonium et le fluorure de tétradécyl-diméthyl-benzyl-ammonium), le nonoxynol 9 et le p-menthanylphényl polyoxyéthylène éther.

A titre de produit phytosanitaire utilisable dans le cadre de la présente invention, on peut notamment citer l'huile de Neem (extraite de *Azadirachta indica*)*.*

L'agent anti-infectieux selon l'invention peut être d'origine chimique (comme c'est le cas pour les antibiotiques, les fongicides et les virucides), ou d'origine végétale ou naturelle.

Par produit d'origine chimique, on entend au sens de la présente invention tous les produits de synthèse totale ou partielle.

Par produit d'origine végétale, on entend au sens de la présente invention le produit total de la plante ou tout type d'extrait de plante, quelle qu'en soit la méthode d'extraction ou de production.

Par produit d'origine naturelle, on entend au sens de la présente invention un produit qui n'est ni d'origine chimique, ni d'origine végétale, mais d'origine animale, comme la Propolis qui est un produit naturel fabriqué par les abeilles.

A titre de produits d'origine végétale utilisables dans le cadre de la présente invention comme agent anti-infectieux, on citera plus particulièrement l'extrait de pépins de pamplemousse, les huiles essentielles, les macérats, les teintures mères, les extraits secs, les extraits aqueux, et les extraits totaux de plantes, et tout autre type d'extrait quelle qu'en soit la méthode d'extraction ou de production.

A titre de produit d'origine naturelle utilisable dans le cadre de la présente invention comme agent anti-infectieux, on citera plus particulièrement la Propolis, qui est un complexe fabriqué par les abeilles à partir de leurs sécrétions et d'une série de substances résineuses, gommeuses et balsamiques.

La composition selon l'invention peut être utilisée dans les domaines de la désinfection de surface, des produits d'hygiène ou des produits cosmétiques dans la mesure où il incorpore un agent anti-infectieux, aussi bien que des produits phytosanitaires et des agents conservateurs alimentaires.

La composition selon l'invention peut donc comprendre en outre un ou plusieurs ingrédients (ou additifs) classiquement utilisés dans les domaines concernés. Les quantités de ces différents ingrédients additionnels sont celles classiquement utilisées dans les domaines concernés.

Bien entendu, l'homme de l'art veillera à choisir le ou les éventuels composés à ajouter à la composition selon l'invention (en fonction en particulier de l'utilisation ou application envisagées), ainsi que leur concentration, de manière à ce que les propriétés avantageuses attachées intrinsèquement, ne soient pas, ou substantiellement pas altérées par l'addition envisagée. En particulier, ce(s) composé(s) additionnel(s) ne devra (devront) pas nuire aux propriétés avantageuses de l'agent anti-infectieux d'une part, et de l'agent dispersant et de l'agent activateur d'autre part.

Ainsi, la présente invention a ainsi pour objet l'utilisation de la composition selon l'invention dans un produit cosmétique ou d'hygiène corporelle.

La composition selon l'invention peut aussi être utilisée à titre d'excipient dans un produit phytosanitaire.

Plus généralement, la composition selon l'invention peut être appliquée au corps humain ou animal dans son ensemble, ou sur les organismes végétaux, ou sur les surfaces inertes.

Enfin, la présente invention a encore pour objet l'utilisation, dans une composition inhibitrice ou destructrice d'au moins un être vivant ou unicellulaire comportant au moins un agent anti-infectieux, de l'association d'au moins un agent activateur comportant au moins un élément métallique à raison d'au plus 20 mg/L dans ladite composition et d'un agent dispersant liposomique, ou micellaire, ou peptidique, ou sous forme de micro-émulsion ou de nano-émulsion, ou encore sous forme de microcapsule, pour augmenter le pouvoir inhibiteur ou destructeur de l'agent anti-infectieux.

L'utilisation selon l'invention d'une telle association permet l'activation de l'action de l'agent anti-infectieux, qui peut notamment être une action antibiotique, bactéricide, fongicide, ou virucide pouvant s'exercer sur l'ensemble du corps humain ou animal, ou sur des surfaces à désinfecter, ou encore sur des organismes végétaux. L'importance de l'activation peut varier suivant les germes rencontrés mais pas la réalité de l'action.

Plus particulièrement, l'utilisation selon l'invention d'une telle association permet l'activation d'une action bactéricide pour lutter contre un agent infectieux des familles bactériennes. Cette action peut s'appliquer à la totalité des familles bactériennes : les *cocci* à Gram positif et à Gram négatif, les bacilles à Gram positif et à Gram négatif, les bacilles acido-alcoolo-résistants, les bactéries de forme spiralée, etc. Cette utilisation à des fins bactéricides permet non seulement d'inhiber les résistances bactériennes (par exemple en général les bactéries bêta-lactamase plus deviennent bêta-lactamase moins) par l'augmentation de l'efficacité des principes actifs, mais autorise également la diminution des doses des principes actifs, quels qu'ils soient, en minimisant ainsi leurs possibles effets adverses sur les êtres humains, les animaux, les végétaux et sur l'environnement en général.

En outre, l'utilisation selon l'invention d'une telle association permet l'activation d'une action fongicide, qui peut s'appliquer aux différentes formes de *fungi :* dermatophytes, levures, etc.

Enfin, l'utilisation selon l'invention d'une telle association permet l'activation d'une action spermicide.

L'invention est illustrée plus en détail dans les exemples suivants. Dans les exemples, sauf indication contraire, toutes les quantités sont indiquées en pourcentage volumique, ou en mg/L, ou en µg ou en µL de composition.

### EXEMPLES

### Produits

● Germes :
   ○ *Staphylococcus aureus* ATCC 29213,
   ○ MRSA (*Staphylococcus aureus* résistant à la méticilline),
   ○ *Escherichia coli* ATCC 25922,
   ○ *Pseudomonas aeruginosa* ATCC 27853, et
   ○ *Streptococcus pneumoniae* ATCC 49619.
● Agents anti-infectieux testés :
   ○ l'huile essentielle de thym (*Thymus vulgaris*)*,* désignée dans la suite des essais par HET,
   ○ l'huile essentielle d'eucalyptus (*Eucalyptus globulus*)*,* désignée dans la suite des essais par HEeuc,
   ○ l'huile essentielle de sarriette (*Satureja montana*)*,* désignée dans la suite des essais par SRT,
   ○ l'huile essentielle de clou de Girofle (*Eugenia caryophyllus*)*,*
   ○ l'huile essentielle de lavande (*Lavendula officinalis*)*,*
   ○ l'huile essentielle de niaouli (*Melaleuca quinquenervia*)*,*
   ○ l'huile essentielle d'origan (*Origanum majorana*)*,*
   ○ l'huile essentielle de ravintsara (*Cinnamomum camphora cineoliferum*)*,*
   ○ l'huile essentielle de romarin (*Rosmarinus officinalis*)*,*
   ○ l'huile essentielle de tea tree (*Melaleuca alternifolia*)*.*
   ○ extrait de pépins de pamplemousse (*Citrus paradisi*)*,* désigné dans la suite des essais par PPM,
● Agent dispersant : agent dispersant liposomique commercialisé par Sigene (Suisse), sous la dénomination commerciale Disper® ; il s'agit d'un complexe émulsionnant naturel consistant en un alcoolat de membranes de cellules végétales composé notamment d'alcool, d'eau, d'un extrait d'amandes douces (*Prunus dulcis*)*,* de lécithine, d'acide oléique, de vitamine C et de vitamine E. Cet agent dispersant a été utilisé dans les exemples précités mais il n'est pas spécifique et peut être remplacé par d'autres agents liposomiques, ou micellaires, etc.
● Agents activateurs métalliques :
   ○ Fluorure de lithium (FLi),
   ○ Fluorure de cobalt (F₂Co),
   ○ Fluorure stanneux (F₂Sₙ).

### Matériel et méthode

Dans un premier temps, il s'agit de déterminer quel est le pourcentage d'inhibition de deux souches bactériennes, qui sont chacune introduite dans un *inoculum,* par un principe actif (ou agent anti-infectieux), en présence d'une composition activatrice du pouvoir inhibiteur comportant au moins un agent activateur métallique associé (conformément à la présente invention) ou non à un agent dispersant (conformément à l'art antérieur).

Par pourcentage d'inhibition, on entend au sens de la présente invention le pourcentage (en % vol/vol) de principe actif nécessaire pour inhiber 100 µL *d'inoculum.*

Dans un deuxième temps, afin d'affiner les résultats, il s'agit de déterminer quelle est la concentration minimale inhibitrice (CMI) d'un principe actif (ou agent anti-infectieux) vis à vis de cinq souches bactériennes, chacune introduite dans un *inoculum,* en présence d'une composition activatrice du pouvoir inhibiteur comportant au moins un agent activateur métallique associé à un agent dispersant, conformément à la présente invention.

Par concentration minimale inhibitrice (CMI), on entend au sens de la présente invention la concentration minimale (en % vol/vol et µL) de principe actif nécessaire pour inhiber 100 µL *d'inoculum.*

Pour déterminer le pourcentage d'inhibition, on réalise, pour chaque exemple, les compositions suivantes :
● un échantillon de contrôle positif (désigné dans les tableaux d'essais par la lettre B), qui contient uniquement *l'inoculum* (100 µL), dans le milieu de culture stérile des germes (100 µL).
● un échantillon de contrôle négatif (désigné dans les tableaux d'essais par la lettre M), qui contient uniquement le milieu de culture stérile des germes (200 µL),
● une première composition inhibitrice comparative CCi, contenant 100 µL de principe actif (i désignant un nombre entier), à laquelle on ajoute 100 µL *d'inoculum,*
● une deuxième composition inhibitrice comparative CCᵢ₊₁, contenant 50 µL de principe actif et 50 µL d'agent activateur métallique (volume total de 100 µL), à laquelle on ajoute 100 µL *d'inoculum,*
● une deuxième composition inhibitrice comparative CCᵢ₊₂, contenant 33,3 µL de principe actif et 66,6 µL d'un mélange à parties égales de deux agents activateurs métalliques (volume total de 100 µL), à laquelle on ajoute 100 µL *d'inoculum,* et
● une ou plusieurs compositions inhibitrices conformément à la présente invention, Ci (i désignant un nombre entier), contenant 100 µL d'un mélange de principe actif, d'activateur métallique et d'agent dispersant, en respectant un volume total de 100 µL, à laquelle on ajoute 100 µL d'*inoculum.*

Pour déterminer la CMI, on réalise, pour chaque exemple, les compositions suivantes :
● un échantillon de contrôle positif (désigné dans les tableaux d'essais par la lettre B), qui contient uniquement l'*inoculum* (100 µL), dans le milieu de culture stérile des germes (100 µL),
● un échantillon de contrôle négatif (désigné dans les tableaux d'essais par la lettre M), qui contient uniquement le milieu de culture stérile des germes (200 µL), et
● une ou plusieurs compositions inhibitrices conformément à la présente invention, Ci (i désignant un nombre entier), contenant 100 µL d'un mélange de principe actif, d'activateur métallique et d'agent dispersant, à laquelle on ajoute 100 µL d'*inoculum.*

Les essais ont été réalisés avec les cinq souches bactériennes mentionnées ci-dessus selon la méthode de micro-dilution en milieu liquide décrite par le CLSI (Clinical and Laboratory Standards Institute) avec adaptation si nécessaire. La méthode peut être résumée de la façon suivante :
- Mettre 100 µL de milieu de culture stérile dans les puits de contrôle positif de la plaque de 96 puits à fond rond.
- Ajouter à chaque puits approprié de la plaque de 96 puits :
   1. 100 µL d'un agent anti-infectieux, ou
      100 µL d'un mélange à parties égales d'un agent anti-infectieux et d'un ou plusieurs agents activateurs, ou
      10 µL d'un agent anti-infectieux et 90 µL d'agent dispersant, ou
      10 µL d'un mélange à parties égales d'un agent anti-infectieux et d'un ou plusieurs agents activateurs et 90 µL d'agent dispersant,
         en respectant un volume total de 100 µL, sauf dans les puits de contrôle positif et de contrôle négatif.
   2. 100 µL *d'inoculum* de concentration finale de 3 - 5 x 10 ⁵ UFC/mL (unités formant colonies par millilitre), sauf dans les puits de contrôle négatif.
- Mettre 200 µL de milieu de culture stérile dans les puits de contrôle négatifs de la plaque de 96 puits.
- Couvrir la plaque et incuber à 37° C pendant 18 à 24 heures.
- Lire les résultats à l'aide d'un miroir-lecteur ou d'un spectrophotomètre à longueur d'onde de 620 nm.

Le seuil de sensibilité n'est pas disponible dans le guide de CLSI (Clinical and Laboratory Standards Institute). Ces essais ont été réalisés au Laboratoire de microbiologie médicale du Centre hospitalier universitaire de Montréal de l'Hôpital Hôtel Dieu, Unité de Recherche et Développement, Montréal, Canada.

### EXEMPLE 1 : activation de l'activité inhibitrice de l'huile essentielle de thym (Thymus vulgaris) (HET)

Outre les compositions de contrôle (positif B et négatif M) précédemment décrites, les compositions suivantes ont été préparées afin de mettre en évidence l'action d'agent(s) activateur(s) métallique(s) seuls ou combinés avec un agent dispersant liposomique :
● une première composition inhibitrice comparative CC₁, contenant 100 µL de HET,
● une deuxième composition inhibitrice comparative CC₂, contenant 50 µL de HET et 50 µL de FLi, soit un volume total de 100 µL,
● une troisième composition comparative CC₃, contenant 33,3 µL de HET et 33,3 µL de FLi + 33,3 µL de F₂Sn, soit un volume total de 100 µL,
● une quatrième composition inhibitrice comparative CC₄, contenant, pour un volume total de 100 µL :
   ○ 10 µL de HET, et
   ○ 90 µL de Disper®,
● une première composition inhibitrice C₁ conformément à l'invention, contenant, pour un volume total de 100 µL :
   ○ 10 µL du mélange suivant : HET (5 µL) + FLi (5 µL), et
   ○ 90 µL de Disper®,
● une deuxième composition inhibitrice C₂ conformément à l'invention, contenant, pour un volume total de 100 µL :
   ○ 10 µL du mélange suivant : HET (3,33 µL) + FLi (3,33 µL) + F₂Sn (3,33 µL), et
   ○ 90 µL de Disper®.

On ajoute 100 µL d'*inoculum* à chacune des compositions décrites ci-dessus.

La concentration finale de FLi dans le milieu, c'est-à-dire dans les 200 µL de chaque puits approprié, est de 8 mg/L. Il y a donc 1,6 µg de FLi dans ces 200 µL. La concentration finale de F₂Sn dans ce milieu est de 5 mg/L, c'est-à-dire qu'il y a 1 µg de F₂Sn dans les 200 µL de chaque puits approprié.

Pour chacune de ces compositions, on a déterminé le pourcentage d'inhibition de 2 souches bactériennes par le principe actif (dans cet exemple, l'huile essentielle de thym). Les résultats expérimentaux de ces essais sont présentés dans le tableau 1 ci-après.

**Tableau 1**

| **Souches bactériennes** | **Contrôle négatif** | **Contrôle positif** | **Pourcentage (%) d'inhibition des souches bactériennes par HET seule ou associée au FLi (8mg/L) ¹ ou au FLi (8 mg/L)¹ + F₂Sn (5 mg/L) ¹, avec ou sans Disper® ²** | | |
|---|---|---|---|---|---|
| (100 µL d' *inoculum*) | | | **CC₁** | **CC₂** | **CC₃** |
| | **M⁴** | **B⁵** | **HET** | **HET** | **HET** |
| | | | (100 µL =50%)³ | (50 µL=25%)³ | (33,3 µL = 16,6 %) ³ |
| | | | | + **FLi** | + **FLi** |
| | | | | (50 µL=25%)³ | (33,3 µL=16,6%) ³ |
| | | | | | + **F₂Sn** |
| | | | | | (33,3 µL=16,6%) ³ |
| *Staphylococcus aureus* ATCC 29213 | ― | + | 79 | 93 | 92 |
| *MRSA* | ― | + | 79 | 93 | 93 |

| **Souches bactériennes** | **Contrôle négatif** | **Contrôle positif** | **Pourcentage (%) d'inhibition des souches bactériennes par HET seule ou associée au FLi (8mg/L) ¹ ou au FLi (8 mg/L) ¹** + F**₂Sn (5 mg/L) ¹, avec ou sans Disper® ²** | | |
|---|---|---|---|---|---|
| (100 µLd' *inoculum*) | | | **CC₄** | **C₁** | **C₂** |
| | **M⁴** | **B⁵** | **HET** | **HET** | **HET** |
| | | | (10 µL = 5 %) ³ | (5 µL = 2,5 %) ³ | (3,33 µL = 1,66 %) ³ |
| | | | + **Disper®** | + **FLi** | + **FLi** |
| | | | (90 µL =45%)³ | (5 µL = 2,5 %) ³ | (3,33 µL = 1,66 %) ³ |
| | | | | + **Disper®** | + **F₂Sn** |
| | | | | (90 µL = 45 %) ³ | (3,33 µL = 1,66 %) ³ |
| | | | | | + **Disper®** |
| | | | | | (90 µL = 45 %) ³ |
| *Staphylococcus aureus* ATCC 29213 | ― | + | 85 | 89 | 87 |
| *MRSA* | ― | + | 81 | 87 | 85 |

| | | | | | |
|---|---|---|---|---|---|
| ¹ Concentrations finales de FLi et F₂Sn dans le milieu, c'est-à-dire 1,6 µg de FLi ± 1 µg de F₂Sn dans les 200 µL de chaque puits approprié. ² [(HET) ou (HET + FLi) ou (HET + FLi + F₂Sn)] + [Disper] [10 : 90]. ³ Pourcentage de chaque produit dans les 200 µL de chaque puits approprié. ⁴ Contrôle négatif (milieu de culture stérile) : pas de croissance bactérienne. ⁵ Contrôle positif (*inoculum*) *:* croissance bactérienne. | | | | | |

Les résultats expérimentaux présentés dans le tableau 1 montrent que les compositions inhibitrices selon l'invention permettent d'inhiber les bactéries avec des doses des principes actifs beaucoup plus faibles (1,66 % ou 2,5 % dans l'exemple 1) que celles nécessaires lorsque les principes actifs sont utilisés seuls (50 % dans cet exemple 1) ou lorsque les principes actifs sont associés seulement à des agents activateurs métalliques (sans agent dispersant, par exemple du type liposomique), comme dans l'art antérieur (25 % dans cet exemple 1).

La composition C₂ selon l'invention contient 30 fois moins d'huile essentielle de thym que la composition comparative CC₁, contenant le même principe actif sans activateur ni dispersant, et possède un pouvoir inhibiteur supérieur (85 % ou 87 % au lieu de 79 %).

Pour le germe *Staphylococcus aureus* ATCC 29213, l'adjonction de FLi à l'huile essentielle de thym (composition CC₂) a permis d'augmenter de 79 % à 93 % le pouvoir d'inhibition de l'huile essentielle de thym, tout en diminuant de moitié le pourcentage d'huile essentielle de thym utilisée qui passe de 50 % à 25 %. L'adjonction de F₂Sn au FLi (composition CC₃) n'augmente pas directement le taux d'inhibition. Mais, pour un résultat peu différent (92 % d'inhibition au lieu de 93 %), elle permet de diminuer le pourcentage d'huile essentielle de thym utilisée à 16,6 %. La conjonction de l'agent dispersant et du FLi dans la composition C₁ selon l'invention augmente l'efficacité de l'huile essentielle de thym (89 % au lieu de 79 %) tout en diminuant de manière importante le pourcentage d'huile essentielle de thym utilisée, qui passe de 50 % à 2,5 %. Dans la composition C₂ selon l'invention, la double activation métallique du FLi et du F₂Sn permet, ajoutée à l'agent dispersant, de réduire à 1,66 % la teneur en huile essentielle de thym. La composition C₂ selon l'invention permet donc de diviser par 30 la quantité d'huile essentielle de thym suffisante pour obtenir un pouvoir inhibiteur supérieur à celui observé avec l'huile essentielle de thym seule, soit 87 % au lieu de 79 %.

Pour le germe *Staphylococcus aureus* résistant à la méticilline (MRSA), les résultats sont similaires à ceux obtenus avec la souche de référence de *Staphylococcus aureus* ATCC 29213. La conjonction de l'agent dispersant et du FLi dans la composition C₁ selon l'invention augmente l'efficacité de l'huile essentielle de thym (87 % au lieu de 79 %) tout en permettant de diminuer le pourcentage d'huile essentielle de thym utilisée : 2,5 % au lieu de 50 %. Dans la composition C₂ selon l'invention, la double activation métallique du FLi et du F₂Sn permet, ajoutée l'agent dispersant, de réduire à 1,66 % la teneur en huile essentielle de thym. La composition C₂ selon l'invention permet donc de diviser par 30 la quantité d'huile essentielle de thym suffisante pour obtenir un pouvoir inhibiteur supérieur à celui observé avec l'huile essentielle de thym seule, soit 85 % au lieu de 79 %.

Ces résultats expérimentaux montrent que les compositions inhibitrices selon l'invention permettent une diminution des doses de principes actifs utilisés, tout en augmentant leur pouvoir inhibiteur.

### EXEMPLE 2: activation de l'activité inhibitrice de l'huile essentielle d'eucalyptus (Eucalyptus globulus) (HEeuc)

Outre les compositions de contrôle (positif B et négatif M) précédemment décrites, les compositions suivantes ont été préparées afin de mettre en évidence l'action d'agent(s) activateur(s) métallique(s) seuls ou combinés avec un agent dispersant liposomique :
● une première composition inhibitrice comparative CC₅, contenant 100 µL de HEeuc,
● une deuxième composition inhibitrice comparative CC₆, contenant 50 µL de HEeuc et 50 µL de FLi, soit un volume total de 100 µL,
● une troisième composition comparative CC₇, contenant 33,3 µL de HEeuc et 33,3 µL de FLi + 33,3 µL de F₂Sn, soit un volume total de 100 µL,
● une quatrième composition inhibitrice comparative CC₈, contenant, pour un volume total de 100 µL :
   ○ 10 µL de HEeuc, et
   ○ 90 µL de Disper®,
● une première composition inhibitrice C₃ conformément à l'invention, contenant, pour un volume total de 100 µL :
   ○ 10 µL du mélange suivant : HEeuc (5 µL) + FLi (5 µL), et
   ○ 90 µL de Disper®,
● une deuxième composition inhibitrice C₄ conformément à l'invention, contenant, pour un volume total de 100 µL :
   ○ 10 µL du mélange suivant : HEeuc (3,33 3 µL) + FLi (3,33 3 µL) + F₂Sn (3,33 3 µL), et
   ○ 90 µL de Disper®.

On ajoute 100 µL d'*inoculum* à chacune des compositions décrites ci-dessus.

La concentration finale de FLi dans le milieu, c'est-à-dire dans les 200 µL de chaque puits approprié, est de 8 mg/L. Il y a donc 1,6 µg de FLi dans ces 200 µL. La concentration finale de F₂Sn dans ce milieu est de 5 mg/L, c'est-à-dire qu'il y a 1 µg de F₂Sn dans les 200 µL de chaque puits approprié.

Pour chacune de ces compositions, on a déterminé le pourcentage d'inhibition de 2 souches bactériennes par le principe actif (dans cet exemple l'huile essentielle d'eucalyptus). Les résultats expérimentaux de ces essais sont présentés dans le tableau 2 ci-après.

**Tableau 2**

| **Souches bactériennes** | **Contrôle négatif** | **Contrôle positif** | **Pourcentage (%) d'inhibition des souches bactériennes par HEeuc seule ou associée au FLi (8mg/L) ¹ ou au FLi (8 mg/L) ¹** + F**₂Sn (5 mg/L) ¹, avec ou sans Disper® ²** | | |
|---|---|---|---|---|---|
| (100 µL d' *inoculum*) | | | **CC₅** | **CC₆** | **CC₇** |
| | **M⁴** | **B⁵** | **HET** | **HET** | **HET** |
| | | | (100 µL=50%)³ | (50 µL=25%)³ | (33,3 µL = 16,6 %) ³ |
| | | | | + **FLi**) | + **FLi** |
| | | | | (50 µL=25%)³ | (33,3 µL=16,6%) ³ |
| | | | | | + **F₂Sn** |
| | | | | | (33,3 µL=16,6%) ³ |
| *Staphylococcus aureus* ATCC 29213 | ― | + | 80 | 95 | 92 |
| *MRSA* | ― | + | 80 | 92 | 90 |

| (100 µL d' *inoculum*) | | | **CC₈** | **C₃** | **C₄** |
|---|---|---|---|---|---|
| | **M⁴** | **B⁵** | **HET** | **HET** | **HET** |
| | | | (10 µL= 5 %) ³ | (5 µL = 2,5 %) ³ | (3,33 µL = 1,66 %) ³ |
| | | | + **Disper®** | + **FLi** | + **FLi** |
| | | | (90 µL =45%)³ | (5 µL = 2,5 %) ³ | (3,33 µL = 1,66 %) ³ |
| | | | | + **Disper®** | + **F₂Sn** |
| | | | | (90 µL = 45 %) ³ | (3,33 µL = 1,66 %) ³ |
| | | | | | + **Disper®** |
| | | | | | (90 µL = 45 %) ³ |
| *Staphylococcus aureus* ATCC 29213 | ― | + | 84 | 94 | 90 |
| *MRSA* | - | + | 82 | 91 | 86 |

| | | | | | |
|---|---|---|---|---|---|
| ¹ Concentrations finales de FLi et F₂Sn dans le milieu, c'est-à-dire 1,6 µg de FLi ± 1 µg de F₂Sn dans les 200 µL de chaque puits approprié. ² [(HEeuc) ou (HEeuc + FLi) ou (HEeuc + FLi + F₂Sn)] + [Disper] [10 : 90]. ³ Pourcentage de chaque produit dans les 200 µL de chaque puits approprié. ⁴ Contrôle négatif (milieu de culture stérile) : pas de croissance bactérienne. ⁵ Contrôle positif (*inoculum*) *:* croissance bactérienne. | | | | | |

Dans cette expérimentation avec l'huile essentielle d'eucallyptus, on observe des résultats comparables à ceux obtenus dans l'exemple 1 avec l'huile essentielle de thym.

Les résultats expérimentaux présentés dans le tableau 2 montrent que les compositions inhibitrices selon l'invention permettent d'inhiber les bactéries avec des doses des principes actifs beaucoup plus faibles (1,66 % ou 2,5 % dans l'exemple 2) que celles nécessaires lorsque les principes actifs sont utilisés seuls (50 % dans cet exemple 2) ou lorsque les principes actifs sont associés seulement à des agents activateurs métalliques (sans agent dispersant, par exemple du type liposomique), comme dans l'art antérieur (25 % dans cet exemple 2).

La composition C₄ selon l'invention contient 30 fois moins d'huile essentielle d'eucalyptus que la composition comparative CC₅, contenant le même principe actif sans activateur ni dispersant, et possède un pouvoir inhibiteur supérieur (86 % ou 90 % au lieu de 80 %).

Pour le germe *Staphylococcus aureus* ATCC 29213, l'adjonction de FLi à l'huile essentielle d'eucalyptus (composition CC₆) a permis d'augmenter de 80 % à 95 % le pouvoir d'inhibition de l'huile essentielle d'eucalyptus, tout en diminuant de moitié le pourcentage d'huile essentielle d'eucalyptus utilisée qui passe de 50 % à 25 %. L'adjonction de F₂Sn au FLi (composition CC₇) n'augmente pas directement le taux d'inhibition. Mais, pour un résultat peu différent (92 % d'inhibition au lieu de 95 %), il permet de diminuer le pourcentage de l'huile essentielle d'eucalyptus utilisée à 16,6 %. La conjonction de l'agent dispersant et du FLi dans la composition C₃ selon l'invention augmente l'efficacité de l'huile essentielle d'eucalyptus (94 % au lieu de 80 %) tout en diminuant de manière importante le pourcentage d'huile essentielle d'eucalyptus utilisée, qui passe de 50 % à 2,5 %.
Dans la composition C₄ selon l'invention, la double activation métallique du FLi et du F₂Sn permet, ajoutée à l'agent dispersant, de réduire à 1,66 % la teneur en huile essentielle d'eucalyptus. La composition C₄ selon l'invention permet donc de diviser par 30 la quantité d'huile essentielle d'eucalyptus suffisante pour obtenir un pouvoir inhibiteur supérieur à celui observé avec l'huile essentielle d'eucalyptus seule, soit 90 % au lieu de 80 %.

Pour le germe *Staphylococcus aureus* résistant à la méticilline (MRSA), les résultats sont similaires à ceux obtenus avec la souche de référence de *Staphylococcus aureus* ATCC 29213, comme dans l'exemple 1 avec l'huile essentielle d'eucalyptus. La conjonction de l'agent dispersant et du FLi dans la composition C₃ selon l'invention augmente l'efficacité de l'huile essentielle d'eucalyptus (91 % au lieu de 80 %) tout en permettant de diminuer le pourcentage d'huile essentielle d'eucalyptus utilisée : 2,5 % au lieu de 50 %. Dans la composition C₄ selon l'invention, la double activation métallique du FLi et du F₂Sn permet, ajoutée à l'agent dispersant, de réduire à 1,66 % la teneur en huile essentielle d'eucalyptus. La composition C₄ selon l'invention permet donc de diviser par 30 la quantité d'huile essentielle d'eucalyptus suffisante pour obtenir un pouvoir inhibiteur supérieur à celui observé avec l'huile essentielle d'eucalyptus seule, soit 86 % au lieu de 80 %.

Ces résultats expérimentaux montrent que les compositions inhibitrices selon l'invention permettent une diminution des doses de principes actifs utilisés, tout en augmentant leur pouvoir inhibiteur.

### EXEMPLE 3 : activation de l'activité inhibitrice de l'huile essentielle de sarriette (Satureja montana) (SRT)

Outre les compositions de contrôle (positif B et négatif M) précédemment décrites, les compositions suivantes ont été préparées afin de mettre en évidence l'action d'agent(s) activateur(s) métallique(s) seuls ou combinés avec un agent dispersant liposomique :
● une première composition inhibitrice C₅ conformément à l'invention, contenant, pour un volume total de 100 µL :
   ○ 10 µL du mélange suivant : SRT (3,33 µL) + FLi (3,33 µL) + F₂Sn (3,33 µL), et
   ○ 90 µL de Disper®,
● une deuxième composition inhibitrice C₆ conformément à l'invention, contenant, pour un volume total de 100 µL :
   ○ 10 µL du mélange suivant : SRT (3,33 µL) + FLi (3,33 µL) + F₂Co (3,33 µL), et
   ○ 90 µL de Disper®.

On ajoute 100 µL d'*inoculum* à chacune des compositions décrites ci-dessus. La concentration finale de FLi dans le milieu, c'est-à-dire dans les 200 µL de chaque puits approprié, est de 8 mg/L. Il y a donc 1,6 µg de FLi dans ces 200 µL. Les concentrations finales de F₂Sn et de F₂Co dans ce milieu sont de 5 mg/L et 4 mg/L respectivement, c'est-à-dire qu'il y a 1 µg de F₂Sn ou 0,8 µg de F₂Co dans les 200 µL de chaque puits approprié.

Pour chacune de ces compositions, on a déterminé la CMI du principe actif (dans cet exemple l'huile essentielle de sariette) pour 5 germes. Les résultats expérimentaux de ces essais sont présentés dans le tableau 3 ci-après.

**Tableau 3**

| **Souches bactériennes** | **Contrôle négatif** | **Contrôle positif** | **CMI (% v/v) des souches bactériennes par SRT associée au FLi (8 mg/L) ¹ + F₂Sn (5 mg/L) , ¹ ou au FLi (8 mg/L)¹ + F₂Co (4 mg/L) ¹, avec Disper® ²** | |
|---|---|---|---|---|
| (100 µL d'inoculum) | | | **C₅** | **C₆** |
| | | | **SRT** (3,33 µL= 1,66%) ³ | **SRT** (3,33 µL = 1,66 %) ³ |
| | **M⁴** | **B⁵** | + **FLi** (3,33 µL= 1,66 %)³ | + **FLi** (3,33 µL = 1,66 %)³ |
| | | | + **F₂Sn** (3,33 µL= 1,66 %)³ | + **F₂Co** (3,33 µL=1,66 %)³ |
| | | | + **Disper®** (90 µL= 45 %)³ | + **Disper®** (90 µL= 45 %)³ |
| *Staphylococcus aureus* ATCC 29213 | ― | + | 0,025 | 0,05 |
| *MRSA* | ― | + | 0,05 | 0,1 |
| *Pseudomonas aeruginosa* ATCC 27853 | ― | + | 0,05 | 0,1 |
| *Escherichia coli* ATCC 25922 | ― | + | 0,1 | 0,1 |
| *Streptococcus pneumomae* ATCC 49619 | ― | + | 0,1 | 0,1 |

| | | | | |
|---|---|---|---|---|
| ¹ Concentrations finales de FLi, F₂Sn et F₂Co dans le milieu, c'est-à-dire 1,6 µg de FLi + 1 µg de F₂Sn ou 1,6 µg de FLi + 0,8 µg de F₂Co dans les 200 µL de chaque puits approprié. ² [(SRT + FLi + F₂Sn) ou (SRT + FLi + F₂Co)] + [Disper] [10 : 90]. ³ Pourcentage de chaque produit dans les 200 µL de chaque puits approprié. ⁴ Contrôle négatif (milieu de culture stérile) : pas de croissance bactérienne. ⁵ Contrôle positif (*inoculum*) *:* croissance bactérienne. | | | | |

Les résultats expérimentaux présentés dans le tableau 3 montrent que les compositions inhibitrices selon l'invention permettent d'inhiber les bactéries avec des doses de principes actifs beaucoup plus faibles que celles nécessaires lorsque les compositions inhibitrices ne comprennent que des agents activateurs métalliques (sans agent dispersant, par exemple du type liposomique). Dans les compositions C₅ et C₆ selon l'invention, la double activation métallique du FLi et du F₂Sn et celle du FLi et du F₂Co permettent, ajoutées à l'agent dispersant, de réduire à 1,66 % la teneur en huile essentielle de sarriette, comme dans les exemples 1 et 2 pour les autres huiles essentielles testées.

Pour les germes *Streptococcus pneumoniae* ATCC 49619 et *Escherichia coli* ATCC 25922, l'effet des deux compositions selon l'invention C₅ et C₆ est équivalent en termes de CMI.

Par contre, pour ce qui concerne les germes *Staphylococcus aureus* ATCC 29213, MRSA *(Staphylococcus aureus* résistant à la méticilline), et *Pseudomonas aeruginosa* ATCC 27853, il faut noter que le fluorure stanneux a un effet catalytique supérieur à celui du fluorure de cobalt lorsque chacun de ces fluorures est associé au fluorure de lithium et au Disper®. Cet effet catalytique amélioré se traduit par une CMI plus faible avec la composition C₅ à base de fluorure stanneux.

### EXEMPLE 4 : activation de l'activité inhibitrice de l'huile essentielle de Thym (Thymus vulgaris) (HET)

Outre les compositions de contrôle (positif B et négatif M) précédemment décrites, les compositions suivantes ont été préparées afin de mettre en évidence l'action d'agent(s) activateur(s) métallique(s) seuls ou combinés avec un agent dispersant liposomique :
● une première composition inhibitrice C₇ conformément à l'invention, contenant, pour un volume total de 100 µL :
   ○ 10 µL du mélange suivant : HET (3,33 3 µL) + FLi (3,3 3 µ L) + F₂Sn (3,33 3 µL), et
   ○ 90 µL de Disper®,
● une deuxième composition inhibitrice C₈ conformément à l'invention, contenant, pour un volume total de 100 µL :
   ○ 10 µL du mélange suivant : HET (3,33 3 µL) + FLi (3,33 µ L) + F₂Co (3,33 3 µL), et
   ○ 90 µL de Disper®.

On ajoute 100 µL d'*inoculum* à chacune des compositions décrites ci-dessus. La concentration finale de FLi dans le milieu, c'est-à-dire dans les 200 µL de chaque puits approprié, est de 8 mg/L. Il y a donc 1,6 µg de FLi dans ces 200 µL. Les concentrations finales de F₂Sn et de F₂Co dans ce milieu sont de 5 mg/L et 4 mg/L respectivement, c'est-à-dire qu'il y a 1 µg de F₂Sn ou 0,8 µg de F₂Co dans les 200 µL de chaque puits approprié.

Pour chacune de ces compositions, on détermine la CMI du principe actif pour 5 germes. Les résultats expérimentaux de ces essais sont présentés dans le tableau 4 ci-après.

**Tableau 4**

| **Souches bactériennes** | **Contrôle négatif** | **Contrôle positif** | **CMI (% v/v) des souches bactériennes par HET associée au FLi (8 mg/L)¹ + F₂Sn (5 mg/L)¹, ou au FLi (8 mg/L)¹ + F₂Co (4 mg/L)¹, avec Disper® ²** | |
|---|---|---|---|---|
| (100 µL d'inoculum) | | | **C₇** | **C₈** |
| | | | **HET** (3,33 µL = 1,66 %)³ | **HET** (3,33 µL = 1,66 %)³ |
| | **M⁴** | **B⁵** | + **FLi** (3,33 µL 1,66 %)³ | **+ FLi** (3,33 µL 1,66 %)³ |
| | | | + **F₂Sn** (3,33 µL= 1,66 %)³ | + **F₂Co** (3,33 µL=1,66 %)³ |
| | | | + **Disper®** (90 µL= 45 %)³ | + **Disper**® (90 µL= 45 %)³ |
| *Staphylococcus aureus* ATCC 29213 | ― | + | 0,05 | 0,1 |
| *MRSA* | ― | + | 0,2 | 0,1 |
| *Pseudomonas aeruginosa* ATCC 27853 | ― | + | 0,05 | 0,1 |
| *Escherichia coli* ATCC 25922 | ― | + | 0,05 | 0,05 |
| *Streptococcus pneumoniae* ATCC 49619 | ― | + | 0,1 | 0,05 |

| | | | | |
|---|---|---|---|---|
| ¹ Concentrations finales de FLi, F₂Sn et F₂Co dans le milieu, c'est-à-dire 1,6 µg de FLi + 1µg de F₂Sn ou 1,6 µg de FLi + 0,8 µg de F₂Co dans les 200 µL de chaque puits approprié. ² [(HET + FLi + F₂Sn) ou (HET + FLi + F₂Co)] + [Disper] [10 : 90]. ³ Pourcentage de chaque produit dans les 200 µL de chaque puits approprié. ⁴ Contrôle négatif (milieu de culture stérile) : pas de croissance bactérienne. ⁵ Contrôle positif *(inoculum) :* croissance bactérienne. | | | | |

De même que dans les exemples 1 à 3, les résultats expérimentaux présentés dans le tableau 4 montrent que les compositions inhibitrices selon l'invention permettent d'inhiber les bactéries avec des doses de principes actifs beaucoup plus faibles que celles nécessaires lorsque les compositions inhibitrices ne comprennent que des agents activateurs métalliques (sans agent dispersant du type liposomique ou autre).

Dans les compositions C₇ et C₈ selon l'invention, la double activation métallique du FLi et du F₂Sn et celle du FLi et du F₂Co permettent, ajoutées à l'agent dispersant, de réduire à 1,66 % la teneur en huile essentielle de thym, comme dans les exemples précédents pour les autres huiles essentielles testées.

Pour le germe *Escherichia coli* ATCC 25922, l'effet des deux compositions selon l'invention C₇ et C₈ est équivalent en termes de CMI.

Pour les germes MRSA *(Staphylococcus aureus* résistant à la méticilline), et *Streptococcus pneumoniae* ATCC 49619, il faut noter que le fluorure de cobalt a un effet catalytique supérieur à celui du fluorure stanneux lorsqu'ils sont associés au fluorure de lithium et au Disper®. Cet effet catalytique amélioré se traduit par une CMI plus faible avec la composition C₈ à base de fluorure de cobalt.

Par contre, pour les germes *Staphylococcus aureus* ATCC 29213 et *Pseudomonas aeruginosa* ATCC 27853, on observe que le fluorure stanneux a un effet catalytique supérieur à celui du fluorure de cobalt lorsqu'ils sont associés au fluorure de lithium et au Disper®, ce qui se traduit par une CMI plus faible avec la composition C₇ à base de fluorure stanneux.

### EXEMPLES 5 à 12: activation de l'activité inhibitrice des huiles essentielles d'eucalyptus (Eucalyptus globulus), de clou de Girofle (Eugenia caryophyllus), de lavande (Lavendula officinalis), de niaouli (Melaleuca quinquenervia), d'origan (Origanum majorana), de ravintsara (Cinnamomum camphora cineoliferum), de romarin (Rosmarinus officinalis) et de tea tree (Melaleuca alternifolia).

De la même manière qu'aux exemples 3 et 4, ont été testés les effets des compositions inhibitrices suivantes :
● un premier groupe de compositions inhibitrices C₉, C₁₁, C₁₃, C₁₅, C₁₇₉ C₁₉, C₂₁, et C₂₃ conformément à l'invention, contenant, pour un volume total de 100 µL :
   ○ 10 µL du mélange suivant : huile essentielle (3,33 µL) + FLi (3,33 µ L) + F₂Sn (3,33 µL), et
   ○ 90 µL de Disper®,
● un deuxième groupe de compositions inhibitrices C₁₀, C₁₂, C₁₄, C₁₆, C_{18,} C₂₀, C₂₂, et C₂₄ conformément à l'invention, contenant, pour un volume total de 100 µL :
   ○ 10 µL du mélange suivant : huile essentielle (3,33 µL) + FLi (3,33 µ L) + F₂Co (3,33 µL), et
   ○ 90 µL de Disper®.

On ajoute 100 µL d'*inoculum* à chacune des compositions décrites ci-dessus.

La concentration finale de FLi dans le milieu, c'est-à-dire dans les 200 µL de chaque puits approprié, est de 8 mg/L. Il y a donc 1,6 µg de FLi dans ces 200 µL. Les concentrations finales de F₂Sn et de F₂Co dans ce milieu sont de 5 mg/L et 4 mg/L respectivement, c'est-à-dire qu'il y a 1 µg de F₂Sn ou 0,8 µg de F₂Co dans les 200 µL de chaque puits approprié.

Pour chacune de ces compositions, on détermine la CMI du principe actif pour 5 germes. Les résultats expérimentaux de ces essais sont présentés dans le tableau 5 ci-après.

**Tableau 5**

| **CMI (% v/v) des souches bactériennes par des huiles essentielles associées au FLi (8 mg/L)¹ + F₂Sn (5 mg/L)¹, ou au FLi (8 mg/L)¹ + F₂Co (4 mg/L)** ¹, **avec Disper®** | | | | | |
|---|---|---|---|---|---|
| **Produits testés** | **Souches bactériennes** | | | | |
| | *Staphylococ-cus aureus* ATCC 29213 | *MRSA* | *Pseudomonas aeruginosa* ATCC 27853 | *Escherichia coli* ATCC 25922 | *Streptococcus pneumoniae* ATCC 49619 |
| **Huile essentielle d'ORIGAN *(organum majorana)*** | | | | | |
| **C**₉ : FLi + F₂Sn + Disper | 0,2 | 0,025 | 0,4 | 0,8 | 0,2 |
| **C₁₀** : FLi + F₂Co + Disper | 0,4 | 0,1 | 0,4 | 0,8 | 0,1 |

| **Huile essentielle de CLOU DE GIROFLE *(Eugenia caryophyllus)*** | | | | | |
|---|---|---|---|---|---|
| **C₁₁**: FLi + F₂Sn + Disper | 0,8 | 0,2 | 0,4 | 0,4 | 0,05 |
| **C₁₂** FLi + F₂Co + Disper | 0,8 | 0,2 | 0,4 | 0,4 | 0,05 |

| **Huile essentielle de ROMARIN *(Rosmarinus officinalis)*** | | | | | |
|---|---|---|---|---|---|
| C₁₃ : FLi + F₂Sn + Disper | 0,2 | 0,1 | 0,8 | 0,8 | 0,2 |
| C₁₄ : FLi + F₂Co + Disper | 0,2 | 0,05 | 1,6 | 0,8 | 0,2 |

| **Huile essentielle de TEA TREE *(Melaleuca alternifolia)*** | | | | | |
|---|---|---|---|---|---|
| **C₁₅** : FLi + F₂Sn + Disper | 0,1 | 0,05 | 1,6 | 0,8 | 0,4 |
| C₁₆ : FLi + F₂Co + Disper | 0,2 | 0,1 | 0,8 | 0,8 | 0,4 |

| **Huile essentielle de RAVINTSARA *(Cinnamomum camphora cineoliferum)*** | | | | | |
|---|---|---|---|---|---|
| C₁₇: FLi + F₂Sn + Disper | 0,4 | 0,05 | 1,6 | 0,8 | 0,2 |
| **C₁₈** : FLi + F₂Co + Disper | 0,2 | 0,05 | 1,6 | 0,8 | 0,2 |

| **Huile essentielle de LAVANDE** ***(Lavendula officinalis**)* | | | | | |
|---|---|---|---|---|---|
| C₁₉ : FLi + F₂Sn + Disper | 0,8 | 0,4 | >1,6 | 0,4 | 0,2 |
| C₂₀ FLi + F₂Co + Disper | 0,8 | 0,2 | >1,6 | 0,4 | 0,4 |

| **Huile essentielle de NIAOULI *(Melaleuca quinquenervia)*** | | | | | |
|---|---|---|---|---|---|
| C₂₁ : FLi + F₂Sn + Disper | 0,2 | < 0,012 | 1,6 | 1,6 | 0,2 |
| C₂₂ : FLi + F₂Co + Disper | 0,4 | < 0,012 | 1,6 | 1,6 | 0,2 |

| **Huile essentielle d'EUCALYPTUS *(Eucalyptus globulus)*** | | | | | |
|---|---|---|---|---|---|
| **C**₂₃ : FLi + F₂Sn + Disper | 0,8 | 0,2 | 1,6 | 1,6 | 0,2 |
| **C**₂₄ : FLi + F₂C_{O} + Disper | 0,8 | 0,1 | 1,6 | 1,6 | 0,4 |

| | | | | | |
|---|---|---|---|---|---|
| ¹ Concentrations finales de FLi, F₂Sn et F₂Co dans le milieu, c'est-à-dire 1,6 µg de FLi + 1 µg de F₂Sn ou 1,6 de FLi + 0,8 µg de F₂Co dans les 200 µL de chaque puits approprié. ² [(HET + FLi + F₂Sn) ou (HET + FLi + F₂Co)] + [Disper] [10 : 90]. Le pourcentage de chaque produit dans le milieu de culture est le même que pour les tableaux 3 et 4 : 1,66 % de chaque huile essentielle (3,33 µL), 1,66 % de FLi (3,33 µL) + 1,66 % de F₂Sn ou de F₂Co (3,33 µL)t 45 % de Disper® (90 µL). | | | | | |

De même que dans les exemples 1 à 4, les résultats expérimentaux présentés dans le tableau 5 montrent que les compositions inhibitrices selon l'invention permettent d'inhiber les bactéries avec des doses de principes actifs beaucoup plus faibles que celles nécessaires lorsque les compositions inhibitrices ne comprennent que des agents activateurs métalliques (sans agent dispersant, par exemple du type liposomique).

Selon les germes et les huiles essentielles, le fluorure stanneux a un effet catalytique supérieur, équivalent ou inférieur à celui du fluorure de cobalt, lorsqu'il est associé au fluorure de lithium et au Disper® avec les huiles essentielles.

Pour le germe *Escherichia coli* ATCC 25922, par exemple, l'effet des deux compositions selon l'invention Cᵢ et Cᵢ₊₁ est équivalent en termes de CMI avec toutes les huiles essentielles testées.

Pour les deux groupes de compositions selon l'invention C₉ à C₂₄, la double activation métallique du fluorure de lithium et du fluorure stanneux et celle du fluorure de lithium et du fluorure de cobalt permettent, ajoutées à l'agent dispersant, de réduire à 1,66 % la teneur en huiles essentielles, comme dans les exemples précédents, pour tous les germes testés.

### EXEMPLE 13 : activation de l'activité inhibitrice de l'extrait de pépins de pamplemousse (Citrus paradisi) (PPM)

Outre les compositions de contrôle (positif B et négatif M) précédemment décrites, les compositions suivantes ont été préparées afin de mettre en évidence l'action d'agent(s) activateur(s) métallique(s) seuls ou combinés avec un agent dispersant liposomique :
● une première composition inhibitrice comparative CC₉, contenant 100 µL de PPM,
●ne deuxième composition inhibitrice comparative CC₁₀, contenant 50 µL de PPM et 50 µL de FLi, soit un volume total de 100 µL,
● une première composition inhibitrice C₂₅ conformément à l'invention, contenant, pour un volume total de 100 µL :
   ○ 10 µL du mélange suivant : PPM (5 µL) + FLi (5 µL), et
   ○ 90 µL de Disper®,
● une deuxième composition inhibitrice C₂₆ conformément à l'invention, contenant, pour un volume total de 100 µL :
   ○ 10 µL du mélange suivant : PPM (3,33 µL) + FLi (3,33 µL) + F₂Sn (3,33 µL), et
   ○ 90 µL de Disper®,
● une troisième composition inhibitrice C₂₇ conformément à l'invention, contenant, pour un volume total de 100 µL :
   ○ 10 µL du mélange suivant : PPM (3,33 µL) + FLi (3,33 µL) + F₂Co (3,33 µL), et
   ○ 90 µL de Disper®.

On ajoute 100 µL *d'inoculum* à chacune des compositions décrites ci-dessus.

La concentration finale de FLi dans le milieu, c'est-à-dire dans les 200 µL de chaque puits approprié, est de 8 mg/L. Il y a donc 1,6 µg de FLi dans ces 200 µL. Les concentrations finales de F₂Sn et de F₂Co dans ce milieu sont de 5 mg/L et 4 mg/L respectivement, c'est-à-dire qu'il y a 1 µg de F₂Sn ou 0,8 µg de F₂Co dans les 200 µL de chaque puits approprié.

Pour chacune des compositions C₂₅ à C₂₇ on détermine la CMI du principe actif pour 5 germes. Les résultats expérimentaux de ces essais sont présentés dans le tableau 6 ci-après.

**Tableau 6**

| **Souches bactériennes** | **Contrôle négatif** | **Contrôle positif** | **CMI (% v/v et µL) des souches bactériennes par PPM seul ou associé au FLi (8 mg/L)¹, ou au FLi (8 mg/L)¹ + Disper® 2, ou au FLi (8 mg/L)¹ + F₂Sn (5 mg/L)¹ + Disper® ², ou au FLi (8 mg/L)¹ + F₂Co (4 mg/L)¹ + Disper® ²** | |
|---|---|---|---|---|
| (100 µL d'inoculum) | | | **CC₉** | **CC₁₀** |
| | | | **PPM** | **PPM** (50 µL = 25 %) ³ |
| | **M⁴** | **B⁵** | (100 µL=50%)³ | + **FLi** (50 µL = 25 %) ³ |
| *Staphylococcus aureus* | - | + | 50 % | 12,5% |
| ATCC 29213 | | | *100 µL* | *25 µL* |
| | | | 25 % | 6,25 % |
| *MRSA* | - | + | *50 µL* | *12,5 µL* |
| *Pseudomonas aeruginosa* ATCC | - | + | >50% | 6,25 % |
| 27853 | | | *> 100 µL* | *12,* µ*L* |
| *Escherichia coli* | - | + | >50% | 25 % |
| ATCC 25922 | | | > *100 µL* | *50 µL* |
| *Streptococcus pneumoniae* | - | + | 12,5% | 6,25% |
| ATCC 49619 | | | *25 µL* | *12,5 µL* |

| **Souches bactériennes** | **CMI (% v/v et µL) des souches bactériennes par PPM seul ou associé au FLi (8 mg/L)¹, ou au FLi (8 mg/L)¹ + Disper® ², ou au FLi (8 mg/L)¹ + F₂Sn (5 mg/L)¹ + Disper® ², ou au FLi (8 mg/L)¹ + F₂Co (4 mg/L)¹ + Disper® ²** | | | |
|---|---|---|---|---|
| (100 µL d' *inoculum)* | **C₂₅** | | **C₂₆** | **C₂₇** |
| | **PPM** (5 µL = 2,5 %)³ | | **PPM** (3,33 µL = 1,66 %)³ | **PPM** (3,33 µL = 1,66 %)³ |
| | +**FLi (**5µL=2,5%)³ | | + **FLi** (3,33 µL = 1,66 %)³ | + **FLi** (3,33 3 µL = 1,66 %)³ |
| | + **Disper®** (90 µL=45 %)³ | | + **F₂Sn** (3,33 3 µL = 1,66 %)³ | + **F₂Co** (3,33 µL=1,66 %)³ |
| | | | + **Disper®** (90 µL = 45 %)³ | + **Disper®** (90 µL= 45 %)³ |
| *Staphylococcus aureus* | 0,625 % | | 0,4 % | 0,4 % |
| ATCC 29213 | 1*, 25* µ*L* | | *0,8 µL* | *0,8 µL* |
| | 0,31 % | | 0,2 % | 0,2 % |
| *MRSA* | *0,62 µL* | | *0,4 µL* | *0,4 µL* |
| *Pseudomonas aeruginosa* ATCC | 0,625 % | | 0,4 % | 0,4 % |
| 27853 | 1*, 25 µL* | | *0,8 µL* | *0,8 µL* |
| *Escherichia coli* | 2,5 % | | 0,8 % | 0,8 % |
| ATCC 25922 | *5 µL* | | *1,6 µL* | *1,6 µL* |
| *Streptococcus pneumoniae* | 0,625 % | | 0,4 % | 0,4% |
| ATCC 49619 | 1*, 25 µL* | | *0,8 µL* | *0,8 µL* |

| | | | | |
|---|---|---|---|---|
| ¹ Concentrations finales de FLi, F₂Sn et F₂Co dans le milieu, c'est-à-dire 1,6 µg de FLi ou 1,6 µg de FLi + 1 µg de F₂Sn ou 1,6 µg de FLi + 0,8 µg de F₂Co dans les 200 µL de chaque puits approprié. ² [(PPM + FLi) ou (PPM + FLi + F₂Sn) ou (PPM + FLi + F₂Co] + [Disper] [10 : 90]. ³ Pourcentage de chaque produit dans les 200 µL de chaque puits approprié. ⁴ Contrôle négatif (milieu de culture stérile) : pas de croissance bactérienne. ⁵ Contrôle positif *(inoculum) :* croissance bactérienne. | | | | |

De même que dans les exemples précédents, les résultats expérimentaux présentés dans le tableau 6 montrent que, pour chaque germe, les compositions inhibitrices selon l'invention permettent d'inhiber les bactéries avec des doses des principes actifs beaucoup plus faibles que celles nécessaires lorsque les compositions inhibitrices ne comprennent que le principe actif ou le principe actif associé à un agent activateur métallique (donc sans agent dispersant, par exemple du type liposomique).

L'adjonction du fluorure de lithium à l'extrait de pépins de pamplemousse dans la composition CC₁₀ selon l'art antérieur permet d'augmenter le pouvoir inhibiteur de l'extrait de pépins de pamplemousse, dont les CMI sont divisées par 2 ou 4 selon les germes, tout en diminuant le pourcentage d'extrait de pépins de pamplemousse utilisé, qui passe de 50 % à 25 %.

La conjonction de l'agent dispersant au fluorure de lithium dans la composition C₂₅ selon l'invention augmente de manière importante l'efficacité de l'extrait de pépins de pamplemousse dont les CMI sont diminuées soit de 95 %, soit de 98,76 % selon les germes, par rapport à celles de l'extrait de pépins de pamplemousse utilisé seul (composition CC₉). Parallèlement, le pourcentage d'extrait de pépins de pamplemousse employé passe de 50 % à 2,5 %.

Dans les compositions C₂₆ et C₂₇ selon l'invention, la double activation du fluorure de lithium et du fluorure stanneux et celle du fluorure de lithium et du fluorure de cobalt permettent, ajoutées à l'agent dispersant, de diminuer les CMI soit de 96,8 %, soit de 99,2 % selon les germes par rapport à celles de l'extrait de pépins de pamplemousse utilisé seul (composition CC₉). Et le pourcentage d'extrait de pépins de pamplemousse employé est diminué de 50 % à 1,66 %, comme dans les exemples précédents.

Les compositions C₂₆ et C₂₇ selon l'invention permettent donc de diviser par 31,25 (pour *Streptococcus pneumoniae* ATCC 49619) ou par 62,5 (pour *Escherichia coli* ATCC 25922) ou par 125 (pour *Staphylococcus aureus* ATCC 29213, *Staphylococcus aureus* résistant à la méticilline [MRSA] et *Pseudomonas aeruginosa* ATCC 27853) la quantité d'extrait de pépins de pamplemousse suffisante pour inhiber ces bactéries.

## Revendications

1. Composition inhibitrice ou destructrice d'au moins un être vivant ou unicellulaire, qui comprend au moins un agent anti-infectieux et au moins un agent activateur comportant au moins un élément métallique, ladite composition étant **caractérisée en ce qu'**elle comporte en outre conjointement au moins un agent dispersant liposomique ou micellaire ou peptidique, ou sous forme de microémulsion ou de nanoémulsion, et
**en ce que** ledit élément métallique de l'agent activateur est présent dans ladite composition à raison d'au plus 20 mg/L.

2. Composition selon la revendication 1, **caractérisée en ce que** l'agent dispersant est choisi parmi les liposomes naturels et synthétiques, de type anionique ou cationique.

3. Composition selon l'une quelconque des revendications précédentes, **caractérisée en ce que** l'agent activateur comporte un élément métallique unitaire ou une association de plusieurs éléments métalliques unitaires, se présentant sous forme d'un colloïde métallique, ou d'un sel métallique, ou sous forme d'un ion métallique.

4. Composition selon l'une quelconque des revendications précédentes, **caractérisée en ce que** l'agent anti-infectieux est choisi parmi les antibiotiques, les bactéricides, les fongicides, les virucides, les antiparasitaires, les désinfectants de surface, les spermicides, et les phytosanitaires, qu'ils soient d'origine chimique, végétale ou naturelle.

5. Composition selon la revendication 4, **caractérisée en ce que** l'agent anti-infectieux est un antibiotique choisi parmi les pénicillines, les céphalosporines, les cyclines, les aminosides, les macrolides, les sulfamides, les quinolones, et les phénicolés.

6. Composition selon la revendication 4, **caractérisée en ce que** l'agent anti-infectieux est un virucide chimique choisi parmi les ammonium quaternaires, ou un virucide d'origine végétale choisi parmi les huiles essentielles d'origan, de thym, et de sarriette, ou parmi les extraits de plantes, ou un virucide d'origine naturelle.

7. Composition selon la revendication 4, **caractérisée en ce que** l'agent anti-infectieux est un bactéricide chimique choisi parmi les ammonium quaternaires, les biguanides, les carbanilides, les composés phénoliques, les composés chlorés, et le glutaraldehyde.

8. Composition selon la revendication 6 ou 7, **caractérisée en ce que** l'agent anti-infectieux est le fluorure de tétradécyl-diméthyl-benzyl-ammonium, et ladite composition est administrable par injection.

9. Composition selon la revendication 4, **caractérisée en ce que** l'agent anti-infectieux est un spermicide choisi parmi les ammonium quaternaires, le p-menthanylphényl-polyoxyéthylène éther et le nonxynol 9.

10. Composition selon la revendication 4, **caractérisée en ce que** l'agent anti-infectieux est un fongicide chimique choisi parmi l'econazole, le ketoconazole, le ciclopirox olamine, ou un fongicide d'origine végétales choisi parmi les huiles essentielles de géranium rosat *(Pelargonium asperum),* de palmarosa *(Cymbopogon martinis* variété *motia),* et de lavande aspic *(Lavendula latifolia),* ou parmi les extraits de plantes, ou un fongicide d'origine naturelle, et de préférence la propolis.

11. Composition selon la revendication 4, **caractérisée en ce que** l'agent anti-infectieux est un produit phytosanitaire, et de préférence l'huile de neem.

12. Composition selon la revendication 4, **caractérisée en ce que** l'agent anti-infectieux est un produit d'origine végétale choisi parmi les huiles essentielles, les macéras, les teintures mères, les extraits secs, les extraits aqueux, les extraits totaux de plantes, et tout type d'extrait de plantes indépendamment de sa méthode d'extraction ou de production, ou un produit d'origine naturelle, et de préférence la propolis.

13. Utilisation de la composition telle que définie selon l'une quelconque des revendications 1 à 12, dans un produit cosmétique ou d'hygiène dans la mesure où il incorpore un agent anti-infectieux, ou dans un produit phytosanitaire, ou des agents conservateurs alimentaires.

14. Utilisation, dans une composition inhibitrice ou destructrice d'au moins un être vivant ou unicellulaire comportant au moins un agent anti-infectieux, de l'association d'au moins un agent activateur comportant au moins un élément métallique à raison d'au plus 20 mg/L dans ladite composition et d'un agent dispersant liposomique ou micellaire ou peptidique, ou sous forme de nanoémulsion, ou sous forme de microémulsion ou de nanoémulsion, pour augmenter le pouvoir inhibiteur ou destructeur dudit agent anti-infectieux.

15. Utilisation selon la revendication 21 **caractérisée en ce qu'**elle permet l'activation des virucides pour lutter contre l'apparition des virus, l'activation des bactéricides pour lutter contre un agent infectieux des familles bactériennes, l'activation des fongicides pour lutter contre l'apparition *des fungi,* et l'activation des spermicides.

## Revendications modifiées

### Revendications modifiées conformément à la règle 137(2) CBE.

**1.** Composition inhibitrice ou destructrice d'au moins un être vivant ou unicellulaire, qui comprend au moins un agent anti-infectieux et au moins un agent activateur comportant au moins un élément métallique, ladite composition étant **caractérisée en ce qu'**elle comporte en outre conjointement au moins un agent dispersant liposomique ou micellaire ou peptidique, ou sous forme de microémulsion ou de nanoémulsion, et
**en ce que** ledit élément métallique de l'agent activateur est présent dans ladite composition à raison d'au plus 20 mg/L.

**2.** Composition selon la revendication 1, **caractérisée en ce que** l'agent dispersant est choisi parmi les liposomes naturels et synthétiques, de type anionique ou cationique.

**3.** Composition selon l'une quelconque des revendications précédentes, **caractérisée en ce que** l'agent activateur comporte un élément métallique unitaire ou une association de plusieurs éléments métalliques unitaires, se présentant sous forme d'un colloïde métallique, ou d'un sel métallique, ou sous forme d'un ion métallique.

**4.** Composition selon l'une quelconque des revendications précédentes, **caractérisée en ce que** l'agent anti-infectieux est choisi parmi les antibiotiques, les bactéricides, les fongicides, les virucides, les antiparasitaires, les désinfectants de surface, les spermicides, et les phytosanitaires, qu'ils soient d'origine chimique, végétale ou naturelle.

**5.** Composition selon la revendication 4, **caractérisée en ce que** l'agent anti-infectieux est un antibiotique choisi parmi les pénicillines, les céphalosporines, les cyclines, les aminosides, les macrolides, les sulfamides, les quinolones, et les phénicolés.

**6.** Composition selon la revendication 4, **caractérisée en ce que** l'agent anti-infectieux est un virucide chimique choisi parmi les ammonium quaternaires, ou un virucide d'origine végétale choisi parmi les huiles essentielles d'origan, de thym, et de sarriette, ou parmi les extraits de plantes, ou un virucide d'origine animale.

**7.** Composition selon la revendication 4, **caractérisée en ce que** l'agent anti-infectieux est un bactéricide chimique choisi parmi les ammonium quaternaires, les biguanides, les carbanilides, les composés phénoliques, les composés chlorés, et le glutaraldehyde.

**8.** Composition selon la revendication 6 ou 7, **caractérisée en ce que** l'agent anti-infectieux est le fluorure de tétradécyl-diméthyl-benzyl-ammonium, et ladite composition est administrable par injection.

**9.** Composition selon la revendication 4, **caractérisée en ce que** l'agent anti-infectieux est un spermicide choisi parmi les ammonium quaternaires, le p-menthanylphényl-polyoxyéthylène éther et le nonxynol 9.

**10.** Composition selon la revendication 4, **caractérisée en ce que** l'agent anti-infectieux est un fongicide chimique choisi parmi l'econazole, le ketoconazole, le ciclopirox olamine, ou un fongicide d'origine végétales choisi parmi les huiles essentielles de géranium rosat *(Pelargonium asperum),* de palmarosa (*Cymbopogon martinii* variété *motia*), et de lavande aspic *(Lavendula latifolia),* ou parmi les extraits de plantes, ou un fongicide d'origine animale.

**11.** Composition selon la revendication 4, **caractérisée en ce que** l'agent anti-infectieux est un produit phytosanitaire.

**12.** Composition selon la revendication 4, **caractérisée en ce que** l'agent anti-infectieux est un produit d'origine végétale choisi parmi les huiles essentielles, les macéras, les teintures mères, les extraits secs, les extraits aqueux, les extraits totaux de plantes, et tout type d'extrait de plantes indépendamment de sa méthode d'extraction ou de production, ou un produit d'origine naturelle.

**13.** Utilisation de la composition telle que définie selon l'une quelconque des revendications 1 à 12, dans un produit cosmétique ou d'hygiène dans la mesure où il incorpore un agent anti-infectieux, ou dans un produit phytosanitaire, ou des agents conservateurs alimentaires.

**14.** Composition comportant au moins un agent anti-infectieux et l'association d'au moins un agent activateur comportant au moins un élément métallique à raison d'au plus 20 mg/L et d'un agent dispersant liposomique ou micellaire ou peptidique, ou sous forme de nanoémulsion, ou sous forme de microémulsion ou de nanoémulsion, pour son utilisation comme médicament dans le traitement des pathologies infectieuses.

**15.** Composition selon la revendication 14, pour son utilisation comme médicament pour lutter contre l'apparition des virus, des agents infectieux des familles bactériennes, des *fungi* , et pour son utilisation pour activer des spermicides.
